# FASCICULE DE BREVET EUROPEEN

(11) **EP 0 419 387 B1**
(45) Date de publication et mention de la délivrance du brevet: **20.11.1996**
(21) Numéro de dépôt: 90430018.3
(22) Date de dépôt: 20.09.1990
(51) Int. Cl.: A61K 51/02, C07K 5/06

(54) **Nouveaux dérivés hydrophiles, application au diagnostic et à la thérapeutique, kits diagnostiques ou thérapeutiques et réactifs immunologiques**
Hydrophile Derivate, diagnostische und therapeutische Anwendungen, diagnostische oder therapeutische Reagenziensätze und immunologische Reagenzien
Hydrophilic derivatives, diagnostic and therapeutic applications, kits and immunological reagents

(30) Priorité: 21.09.1989 FR 8912622
(43) Date de publication de la demande: 27.03.1991
(73) Titulaire: IMMUNOTECH PARTNERS:Société Anonyme dite, F-13276 MARSEILLE CEDEX 9 (FR)
(72) Inventeur: Barbet, Jacques, F-13009 Marseille (FR); Delaage, Michel, F-13001 Marseille (FR); Gruaz-Guyon, Anne, F-92100 Boulogne (FR); Le Doussal, Jean-Marc, F-13009 Marseille (FR)
(74) Mandataire: Rinuy, Santarelli

(56) Documents cités:
- EP-A- 0 006 792
- EP-A- 0 217 577
- EP-A- 0 251 494
- EP-A- 0 263 046
- EP-A- 0 327 365
- WO-A-83/03679
- WO-A-89/07114
- US-A- 4 479 930

## Description

La présente invention concerne des dérivés utiles notamment comme immuno-réactifs destinés en particulier à cibler des cellules animales en vue de leur visualisation ou de leur destruction, les kits de réactifs les renfermant ainsi que leur application à la visualisation et à la destruction des cellules.

WO 83/03679 décrit des produits de fusion d'un hybridome avec un lymphocyte B ou un autre hybridome, produisant un anticorps monoclonal hybride ayant une double spécificité contre deux déterminants antigéniques différents ainsi que des procédés immuno-diagnostiques et immuno-thérapeutiques utilisant des anticorps ayant une double spécificité, l'une dirigée vers un antigène cible, et l'autre contre un reste qui permet de réaliser un diagnostic ou une thérapie.

EP-A-0.217.577 décrit des complexes anticorps comprenant des agents de diagnostic ou thérapeutiques modifiés en tant qu'haptènes et des anticorps anti-haptènes. L'administration de ces agents complexés avec des anticorps anti-haptènes convenables permet d'obtenir une demi-vie sérique prolongée ainsi qu'une amélioration de la concentration au niveau de leur cible in vivo.

EP-A-0.251.494 décrit un système pour administrer un agent diagnostic ou thérapeutique au niveau d'une cible interne d'un individu comprenant un composé biotinylé contenant l'agent biotiné, une protéine avidinée capable de se localiser de manière sélective au niveau de sa cible, et un agent de clearance capable de réagir avec ladite protéine dans la circulation sanguine.

EP-A-0.006.792 décrit un composé conjugué d'un antigène présentant une fonction carboxylique libre et d'un peptide, ce dernier étant constitué par un produit de condensation entre la tyrosine et un peptide, le groupement tyrosine dudit peptide étant marqué à l'iode radioactif, et son application en radio-immunologie.

EP-A-327.365 décrit une méthode pour modifier la pharmacocinétique d'un haptène utilisable comme agent dit d'imagerie radioactive ou radiothérapeutique ainsi que des compositions comprenant un dérivé d'haptène comprenant un radical benzyl-EDTA.

La demande de brevet francais FR-A-2.604.092 et la demande de brevet européen EP-A-0.263.046 ont décrit des immuno-réactifs destinés à cibler les cellules animales pour leur visualisation ou leur destruction in vivo.

Ces immuno-réactifs consistent en deux types de produits à savoir a) un anticorps monoclonal ou un fragment d'anticorps reconnaissant un type cellulaire, un tissu particulier ou un constituant d'un organisme animal conjugué à un second anticorps ou fragment d'anticorps reconnaissant un groupement hapténique déterminé et b) une molécule constituée d'au moins deux haptènes identiques ou différents reconnus par l'un des anticorps et d'un motif constitué d'un élément de visualisation, ou d'un produit actif ou connu pour pouvoir recevoir un élément de visualisation ou un produit actif destiné à la destruction de la cellule-cible.

Dans ce qui suit, on appellera le produit (b) "sonde".

Lorsque l'on veut réaliser une bonne visualisation d'une cellule-cible, il est souhaitable de réduire au maximum le bruit de fond. De même, étant donné que les principes actifs cytotoxiques possèdent habituellement une certaine toxicité, il est souhaitable de les retrouver seulement au niveau de la cellule-cible à détruire, le plus rapidement possible après leur administration.

C'est pourquoi la présente demande a pour objet des dérivés caractérisés en ce qu'ils comprennent deux haptènes hydrophiles et un groupe effecteur constitué d'un isotope radioactif ou connu pour pouvoir être marqué par un isotope radioactif ou constitué d'un principe actif ou connu pour pouvoir fixer un principe actif. Ces dérivés sont analogues à des produits tels que ceux décrits en b) ci-dessus.

Par "haptène", l'on entend dans le cadre de la présente invention, une molécule capable de se lier spécifiquement à un anticorps au niveau d'un de ses sites de fixation. C'est une molécule qui n'est pas antigénique par elle-même mais peut le devenir par fixation sur une macromolécule. Sa masse moléculaire est généralement inférieure à 1500 daltons.

Par "hydrophiles", l'on entend que le coefficient de partition entre une phase aqueuse neutre (proche des conditions physiologiques) et un solvant organique non totalement miscible à l'eau comme le N-butanol est supérieur à 1 pour le haptène isolé. Cette mesure peut être effectuée, notamment après 2h d'agitation d'une solution d'haptène dans 10 mM Hepes, 150 mM Nacl, pH 7,4 mélangée à une partie égale de butanol.

Les haptènes préférés selon la présente invention auront une masse moléculaire comprise entre 300 et 1500 daltons et comporteront des groupements chimiques polaires. Ces groupements seront notamment des groupes alcools, amines, acides carboxyliques, sulfoniques et phosphoriques, ainsi que leurs esters et amides.

Parmi les dérivés objet de la présente invention, on retient de préférence ceux caractérisés en ce que les haptènes ont une constante de dissociation inférieure ou égale à 10-⁵ M et notamment inférieure ou égale à 10-⁶ M avec un anticorps qui leur est spécifique. Cette constante est mesurée dans les conditions usuelles de pH, température et concentration en sels.

Comme indiqué dans la demande de brevet français précitée, les haptènes peuvent revêtir une grande variété de structures. On retient toutefois plus particulièrement les haptènes qui constituent un groupe chélatant.

Par "chélatant", l'on entend une molécule capable de fixer un atome de métal de manière très stable.

Les groupes chélatants particulièrement intéressants convenant comme haptènes hydrophiles selon la présente invention sont constitués par les acides polyaminocarboxyliques, notamment l'acide diéthylènetriamine-pentaacétique (DTPA) et ses dérivés.

Ces groupes chélatants peuvent contenir ou non un métal, et on retient plus particulièrement ceux contenant de l'indium complexé.

A titre d'haptènes, on retient également les dérivés des aminoacides polaires et particulièrement de l'histidine et les dérivés de l'histamine, tout particulièrement l'histamine-succinyl-glycine. On peut citer également la fluorescéine et ses dérivés et le méthotrexate.

Dans une sonde, les haptènes et le(s) groupe(s) effecteur(s) sont reliés de manière stable par une chaîne de connexion. Celle-ci permet la fixation simultanée sur une même molécule sonde d'au moins deux anticorps spécifiques sur le ou les haptènes et ainsi la formation des complexes particuliers recherchés.

Un des haptènes peut également jouer le rôle d'effecteur ; en particulier un de ses atomes peut être remplacé dans la sonde par un radio-isotope ou alors un principe actif tel que ceux décrits ci-après ; une partie du principe actif peut également constituer le haptène. On peut citer par exemple le DTPA-indium.

L'on entend par "groupement ef fecteur", un groupement chimique responsable de l'effet désiré, consécutivement à l'utilisation de la sonde, sur sa cible dans l'organisme. Le groupement effecteur peut réaliser soit le repérage de sites d'accumulation de la sonde, soit par exemple la destruction de cellules au niveau de sites d'accumulation.

Dans le premier type d'application, le groupement effecteur comportera par exemple un ou plusieurs atomes repérables par des moyens physiques appropriés, comme par exemple des atomes radioactifs émetteurs de rayonnement, notamment gamma. On peut citer également les atomes ou groupements chimiques suffisamment para-magnétiques pour perturber les signaux de résonance magnétique nucléaire.

Dans le second type d'applications, le groupement effecteur pourra comporter un ou plusieurs émetteurs de rayonnement ionisant, par exemple f3 ou a en vue d'une radio-thérapie. Il pourra aussi être constitué d'un ou plusieurs groupes chimiques capables de cytotoxicité par eux-mêmes, comme les agents alkylants, le méthotrexate, les vinca- alcaloïdes, les anthracyclines ou des toxines végétales ou bactériennes comme la ricine ou la toxine diphtérique, ou encore sous l'effet d'un rayonnement extérieur comme les porphyrines.

Des chaînes de connexion préférées sont celles qui permettent une séparation suffisante des haptènes, notamment supérieure à 10 Angströms.

Ces chaînes seront de préférence peu sensibles à l'hydrolyse une fois injectées dans l'organisme. On utilise avantageusement à titre de chaînes de connexion des aminoacides, de préférence de série D, en enchaînement peptidique.

Pour certaines applications particulières, les chaînes de connexion ci-dessus décrites comprennent un groupement phénol et on retient tout particulièrement les chaînes de connexion renfermant de la tyrosine ou constituées de tyrosine.

Parmi les dérivés objets de la présente invention, l'on retient également ceux dont les haptènes sont des glycyl- succinyl-histamine, qui sont couplés à une diamine phénolée, notamment couplés à un peptide renfermant de la tyrosine.

Parmi les dérivés selon la présente invention, on retient tout particulièrement les dérivés de la tyrosine suivants :
- la Na-α-DTPA-tyrosyl-N-ε-DTPA-lysine ;
- la N-α-N-ε-di-(DTPA-glycyl)-lysyl-tyrosine ;
- la N-α-N-ε-di-(histamine-succinyl-glycyl) lysyl-tyrosine de formules respectives :

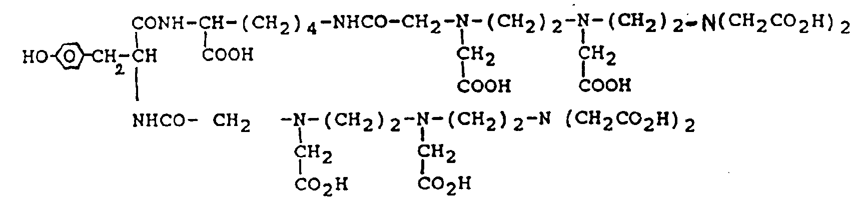

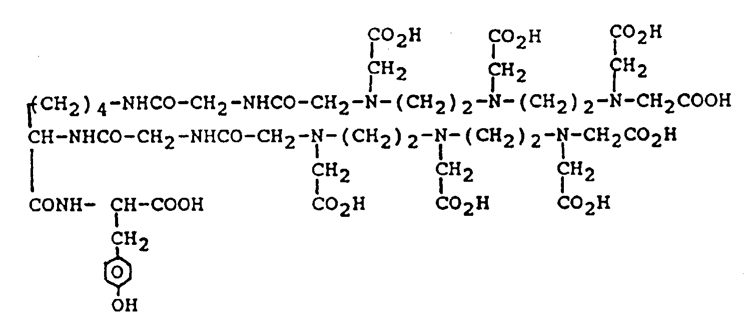

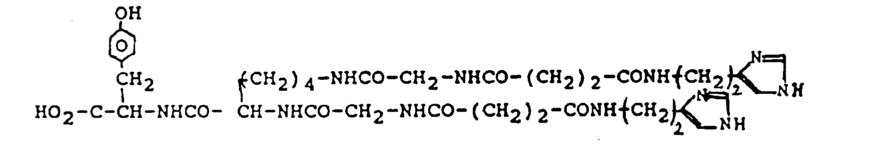

Les dérivés ci-dessus décrits possèdent de remarquables propriétés grâce aux deux groupements hapténiques hydrophiles et à leur groupement effecteur.

C'est pourquoi la présente demande a également pour objet l'application des dérivés tels que définis ci-dessus au diagnostic ou à la thérapeutique.

Pour ces applications, on pourra se reporter notamment à la demande de brevet européen N° 87.430031.2 précitée.

En vue de leur utilisation diagnostique, les dérivés selon la présente invention peuvent être par exemple injectés par voie intraveineuse à l'homme ou à l'animal.

En même temps ou peu avant l'injection de la sonde (par exemple quelques minutes jusqu'à quelques heures avant (de préférence de 12 à 48 heures), on aura injecté un conjugué anticorps à double spécificité composé d'un anticorps notamment monoclonal ou fragment d'anticorps également de préférence monoclonal spécifique pour une cellule ou un composant normal ou pathologique d'un être vivant en particulier de l'homme, couplé à un anticorps, notamment monoclonal ou fragment d'anticorps de préférence spécifique pour le haptène considéré, tel que décrit ci-dessous en tant que produit a), par exemple à la dose de 0,1 mg à 1 g selon la sonde utilisée et de préférence de 1 à 100 mg chez l'homme adulte.

Le dérivé a) peut être utilisé seul ou en mélange (cocktail), chacun ayant une spécificité particulière pour des cellules ou constituants de l'organisme normaux ou pathologiques différents et une spécificité pour le même haptène.

On procède après l'injection des dérivés selon l'invention à la mesure ou la détection grâce aux appareils bien connus dans l'état de la technique, par exemple pour la scintigraphie ou l'imagerie RMN.

Dans le cas d'un diagnostic isotopique on aura par exemple apporté une activité de 0,1 à 100 mCi et plus particulièrement de 3 à 10 mCi.

De même, on peut procéder au traitement localisé d'une affection, par exemple tumorale, en utilisant une sonde selon la présente invention, possédant comme effecteur un principe actif approprié, notamment cytotoxique.

Le taux sera alors limité par la toxicité secondaire du principe actif sur les organes normaux et en particulier sur la moelle osseuse.

Pour une application en radio-immunothérapie, une dose préférée sera comprise par exemple entre 50 mCi et 1 Ci.

Compte tenu de l'utilisation particulière des dérivés selon la présente invention, la présente demande a également pour objet les kits diagnostiques ou thérapeutiques, caractérisés en ce qu'ils renferment au moins un des dérivés selon la présente invention, tels que définis cidessus et un anticorps ou fragment d'anticorps, notamment monoclonal reconnaissant un type cellulaire ou un tissu particulier, conjugué à un deuxième anticorps ou fragment d'anticorps reconnaissant un groupement hapténique dudit dérivé.

La présente demande a enfin pour objet les réactifs immunologiques comprenant un conjugué, composé d'un anticorps ou fragment d'anticorps notamment monoclonal ayant une affinité pour un type cellulaire particulier ou un constituant normal ou pathologique de l'organisme particulier, couplé à un anticorps ou fragment d'anticorps notamment monoclonal ayant une affinité pour un haptène donné, et une molécule synthétique, comprenant au moins deux haptènes correspondant au conjugué ci-dessus et au moins un site, apte au marquage radioactif ou à fixer un principe actif, liés de manière covalente, caractérisés en ce que la molécule synthétique est un dérivé ci-dessus défini.

Lorsque l'on parle de conjugué d'anticorps, on ne préjuge pas d'une méthode de préparation quelconque en ce sens que l'on vise aussi des anticorps bispécifiques encore appelés recombinants, quadromes ou polydomes, par exemple.

Les exemples qui suivent illustrent la présente invention sans toutefois la limiter.

### Exempte 1

### N-a-DTPA-tyrosyl-N-s-DTPA-lysine

On dissout 45 µMole de tyrosyl-lysine dans 600 wl d'eau ajoutée à 45 µMole de triéthylamine et 180 µMole de l'anhydride cyclique du DTPA en solution dans 1 ml de DMSO.

On laisse pendant 16 heures à la température ambiante en maintenant le pH à environ 7 à l'aide de triéthylamine. On purifie par filtration sur gel (colonne Biogel P4). On récupère les fractions correspondant au produit attendu et les purifie sur une colonne échangeuse d'ions (mono-Q pharmacia) et chromatographie liquide haute pression en utilisant une colonne C-18.

On a identifié le produit obtenu par spectrométrie UV (max à 277nm) et spectrométrie de masse (pic moléculaire à 1060).

### Exemple 2

### N-a-N-c-di-(DTPA-glycyl)-lysyl-tyrosine

A partir de 1 g de résine de N-a-BOC-O-benzyl-L-tyrosine, on prépare le N-α-N-E-di-(glycyl)-lysyl-tyrosine classiquement par synthèse en phase solide.

On libère à l'aide d'acide fluorhydrique liquide le produit obtenu et on le purifie par filtration sur gel et chromatographie liquide à haute pression. On fait alors réagir ce peptide avec l'anhydride cyclique du DTPA, comme indiqué ci-dessus à l'exemple 1 ; on purifie le produit obtenu par filtration sur gel, chromatographie sur colonne échangeuse d'ions et chromatographie liquide haute pression et on l'identifie comme décrit ci-dessus ( max à 277 nm, pic moléculaire à 1174).

### Exemple 3

### N-a-N-r-di-(histamine-succinyl-glycyl)-lysyl-tyrosine

On synthétise la N-α-N-s-di-(glycyl)-lysyl-tyrosine sur résine comme indiqué à l'exemple 2. Après déprotection des groupes amino terminaux, on ajoute à la résine 2,5 équivalents d'anhydride succinique dans le DMF et 2,5 équivalents de triéthylamine. On laisse pendant 2 heures à la température ambiante, lave et ajoute 2,6 équivalents d'histamine et de BOP en solution dans le DMF et 3,5 équivalents de diisopropyléthylamine.

On laisse encore incuber, lave et libère le produit désiré ; on purifie par filtration sur gel et par chromatographie liquide haute pression et identifie le produit obtenu par spectrométrie UV (max à 220 nm et à 277 nm) et de masse (pic moléculaire à 810).

### Exemple 4

### Marquage du dérivé de l'exemple 1 à l'indium-111

On prépare une dilution à 0,5 µM du produit de l'exemple 1 dans un tampon à pH 5, 100 mM d'acétate et mM de citrate. On ajoute 0,5 mCi de trichlorure d'indium-111 (dans 50 µl d'acide chlorhydrique 50 mM) à 50 µl de la solution ci-dessus et on laisse incuber pendant 16 heures à 37°C.

On ajoute alors un excès d'InCl₃ non radioactif (10 µl d'une solution 1 mM dans un tampon pH 5 citrate 100 mM) pour saturer les deux groupes chélatants du dérivé de l'exemple 1.

Le dérivé de l'exemple 1 marqué est dilué à l'activité souhaitée juste avant l'injection à l'aide d'une solution 20 mM Hepes, 150 mM HCI, 10 pM EDTA pH 7,4.

### Exemple 5

Radioimmunoscintigraphie de mélanomes humains greffés sur des souris nudes visualisés par des conjugués à double spécificité anti-mélanome anti-DTPA indium et la N-a-DTPA-tyrosyl-N-s-DTPA-lysine marquée à l'indium-111.

On injecte par voie sous-cutanée 3.10⁶ cellules de mélanome humain (A375) dans l'abdomen de souris nudes. Celles-ci développent 3 à 4 semaines plus tard des tumeurs de 0,5 à 2 g. On injecte alors aux souris 2 à 10 µg de conjugué à double spécificité préparé par couplage chimique du fragment F(ab)'₂ d'un anticorps anti mélanome humain (anticorps monoclonal de souris IgG₁, kappa) au fragment F(ab)'₂ réduit d'un anticorps anti-DTPA-indium (anticorps monoclonal de souris IgG,, lambda) au moyen du réactif hétérobifonctionnel, EMCS.

Vingt quatre heures plus tard on injecte aux souris 10 µCi du dérivé de l'exemple 1 marqué a l'indium-111. On prend alors des images à des intervalles de temps choisis en utilisant une gamma caméra Sopha Medical Gammatome 2 équipée d'un collimateur à haute résolution et moyenne énergie, ou alors on tue les animaux et on mesure à l'aide d'un compteur gamma la radioactivité associée à l'indium-111, des principaux organes et de la tumeur.

Quelques minutes après l'injection du dérivé de l'exemple 1 marqué, des tumeurs peuvent déjà être observées sur les images obtenues par gamma caméra. On constate qu'une forte majorité de la radioactivité circulante est éliminée en moins de 24 heures alors que celle fixée sur la tumeur s'établit en quelques heures et reste stable pendant au moins deux jours.

L'accumulation non spécifique de radioactivité a été essentiellement limitée aux reins.

La radioactivité mesurée après 24 heures au niveau des organes et des tumeurs montre un rapport tumeurs/orga- nes élevé (T/O > 3) dans tous les organes, à l'exception des reins (T/O = 0,7).

La fixation tumorale a été spécifique, étant donné que les tumeurs n'ont pas accumulé de quantités significatives de radio-activité lorsque l'on n'a pas injecté de conjugués anticorps à double spécificité ou lorsque le conjugué était spécifique d'un antigène associé à une autre tumeur. Lorsque du DTPA marqué à l'indium-111 (analogue monovalent du dérivé de l'exemple 1 reconnu par l'anticorps anti-indium-DTPA avec une forte affinité) a été injecté à la place du dérivé de l'exemple 1, la radioactivité est excrétée très rapidement et on a observé peu de fixation spécifique.

Quand on a injecté le fragment F(ab)'₂ directement marqué de l'anticorps anti-mélanome, on a observé une fixation spécifique sur la tumeur mais la radioactivité circulante s'est éliminée plus lentement et les rapports tumeurs/orga- nes à 24 heures étaient plus faibles (compris entre 0,4 et 7). La fixation non spécifique dans les reins et le foie a été particulièrement élevée (T/O = 0,4 et 1,5 respectivement).

En conclusion, les dérivés selon la présente invention présentent de remarquables résultats de visualisation.

### Exemple 6

### Tolérance des sondes chez l'animal

Des doses correspondant à 10 nanomoles, soit environ 10000 fois plus que la dose qui est nécessaire pour une radioimmunoscintigraphie, des composés 1 et 2 préalablement complexés avec de l'indium non radioactif et du composé 3 ont été administrées à des souris BALB/c. Ces souris ont ensuite été observées pendant 16 jours dans des conditions normales d'élevage. On n'a constaté aucun signe de toxicité et la prise de poids de ces animaux n'a pas été significativement différente de celle d'un lot d'animaux témoins.

### Exemple 7

Comparaison entre le dérivé de l'exemple 1 marqué à l'indium-111 et le di-(DNP-lys)-DTPA dans le marquage de tumeurs chez la souris.

Les expériences sont conduites comme dans l'exemple 5, mais des souris reçoivent le di-(DNP-lys)-DTPA marqué à l'indium-111 (dérivé 1 de la demande de brevet français 86.13146 (FR-A-2 604 092)) à la place du dérivé de l'exemple 1 décrit ci-dessus. Trois heures après injection de la sonde, les souris sont disséquées et on compte la radioactivité gamma des principaux organes et de la tumeur. On observe que la tumeur a accumulé une dose de radioactivité équivalente A celle obtenue avec le composé de l'exemple 1 de la présente invention. En revanche, l'élimination de l'excès de radioactivité est significativement plus rapide avec le composé de l'exemple 1 de la présente invention, comme l'indique le tableau suivant :

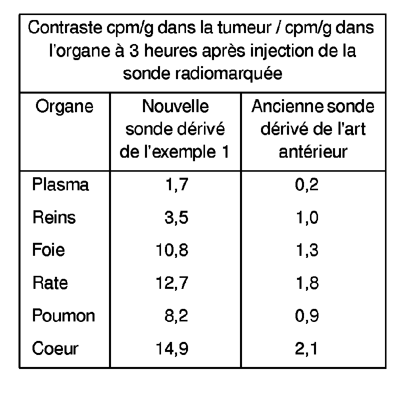

Le contraste tumeur sur organe est donc bien meilleur avec les dérivés de la présente demande qu'avec ceux de l'art antérieur.

### Exemple 8

### N-a-acétyl, N-ε-DPTA, L-lysil-L-tyrosyl-N-r-DPTA-L-lysyl-amide

En opérant comme indiqué à l'exemple 2, on a préparé le produit recherché, identifié comme ci-dessus (max à 277 nm, pic moléculaire à 1228).
Ce dérivé est doué de propriétés très proches de celles du produit de l'exemple 1.

### Exemple 9

### N-α-acétyl, N-ε(histamine-succinyl-glycyl)-L-lysyl-L-tyrosyl-N-ε-(histamine-succinyl-glycyl)-L-lysyl-amide

En opérant comme indiqué à l'exemple 3, on a préparé le produit attendu, identifié comme ci-dessus (max à 220 et 277 nm, et pic moléculaire à 978).

## Revendications (Revendications pour l'(les) Etat(s) contractant(s) suivant(s) : AT, BE, CH, DE, DK, FR, GB, GR, IT, LI, LU, NL, SE)

1. Les dérivés schématisés par la formule I 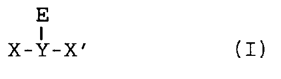 dans laquelle :
- X et X' sont des haptènes, c'est à dire des groupements organiques de masse inférieure à 1 500 daltons capables de se lier à un anticorps avec une constante de dissociation inférieure à 10⁻⁶ mole/l, caractérisés par leur propriété hydrophile définie par un coefficient de partage supérieur à 1 entre une phase aqueuse et un solvant organique en faveur de la phase aqueuse,
- Y est une chaîne de connexion permettant la liaison simultanée sur le même dérivé de formule l d'au moins deux anticorps spécifiques desdits haptènes,
- E est un groupement effecteur, c'est-à-dire apte au marquage radioactif,
- étant entendu que ni X, ni X' ne peut être le benzyl EDTA ou un dérivé du benzyl EDTA, et que la chaîne de connexion ne peut pas être un polymère.

2. Les dérivés selon la revendication 1 caractérisés en ce que le groupement effecteur E coïncide avec un des haptènes X ou X'.

3. Les dérivés selon l'une quelconque des revendications 1 à 2, caractérisés en ce que les haptènes sont des groupes chélatants.

4. Les dérivés selon l'une quelconque des revendications 1 à 3, caractérisés en ce que la chaîne de connexion est un peptide.

5. Les dérivés selon l'une quelconque des revendications 3 ou 4, caractérisés en ce que le groupe chélatant est un dérivé du DTPA.

6. Les dérivés selon l'une quelconque des revendications 1 à 5, caractérisés en ce que la chaîne de connexion comprend un groupement phénol.

7. Les dérivés selon la revendication 6, caractérisés en ce que la chaîne de connexion comprend de la tyrosine ou est constituée de tyrosine.

8. La N-α-DTPA-tyrosyl-N-ε-DTPA-lysine.

9. La N-α-N-ε-di-(DTPA-glycyl)-lysyl-tyrosine.

10. La N-α-N-ε-di-(histamine-succinyl-glycyl)-lysyl-tyrosine.

11. Le N-α-acétyl,N-ε-DTPA,L-lysil-L-tyrosyl-N-ε-DTPA-L-lysyl-amide.

12. Le N-a-acétyl, N-ε-(histamine-succinyl-glycyl)-L-lysyl-L-tyrosyl-N-s-(histamine-succinyl-glycyl)-L-lysyl-amide.

13. Kits diagnostiques ou thérapeutiques, caractérisés en ce qu'ils renferment au moins un des dérivés selon l'une quelconque des revendications 1 à 12 et un anticorps ou fragment d'anticorps, notamment monoclonal reconnaissant un type cellulaire ou un tissu particulier, conjugué à un deuxième anticorps ou fragment d'anticorps reconnaissant un groupement hapténique dudit dérivé.

14. Réactifs immunologiques comprenant :
- un conjugué composé d'un anticorps ou fragment d'anticorps notamment monoclonal ayant une affinité pour un type cellulaire particulier ou un constituant normal ou pathologique de l'organisme particulier, couplé à un anticorps ou fragment d'anticorps notamment monoclonal ayant une affinité pour un haptène donné, et
- une molécule synthétique comprenant au moins deux haptènes correspondant au conjugué ci-dessus et au moins un site, apte au marquage radioactif ou à fixer à un principe actif, liés de manière covalente,
caractérisés en ce que la molécule synthétique est un dérivé défini à l'une des revendications 1 à 12.

15. Les dérivés schématisés par la formule 1 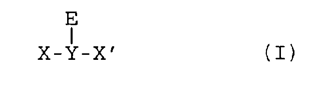 dans laquelle :
- X et X' sont des haptènes, c'est à dire des groupements organiques de masse inférieure à 1 500 daltons capables de se lier à un anticorps avec une constante de dissociation inférieure à 10⁻⁶ mole/l, caractérisés par leur propriété hydrophile définie par un coefficient de partage supérieur à 1 entre une phase aqueuse et un solvant organique en faveur de la phase aqueuse,
- Y est une chaîne de connexion permettant la liaison simultanée sur le même dérivé de formule l d'au moins deux anticorps spécifiques desdits haptènes,
- E est un groupement effecteur, c'est-à-dire apte au marquage radioactif,
- étant entendu que ni X, ni X' ne peut être le benzyl EDTA ou un dérivé du benzyl EDTA, et que la chaîne de connexion ne peut pas être un polymère,
pour leur application dans une méthode de diagnostic ou de traitement du corps humain ou animal.

16. Application des dérivés schématisés par la formule l 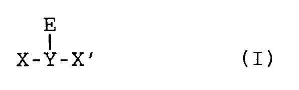 dans laquelle :
X et X' sont des haptènes, c'est à dire des groupements organiques de masse inférieure à 1 500 daltons capables de se lier à un anticorps avec une constante de dissociation inférieure à 10⁻⁶ mole/l, caractérisés par leur propriété hydrophile définie par un coefficient de partage supérieur à 1 entre une phase aqueuse et un solvant organique en faveur de la phase aqueuse,
- Y est une chaîne de connexion permettant la liaison simultanée sur le même dérivé de formule l d'au moins deux anticorps spécifiques desdits haptènes,
E est un groupement effecteur, c'est-à-dire apte au marquage radioactif,
- étant entendu que ni X, ni X' ne peut être le benzyl EDTA ou un dérivé du benzyl EDTA, et que la chaîne de connexion ne peut pas être un polymère,
à l'augmentation du contraste dans la visualisation d'une cellule-cible par utilisation conjointe avec un anticorps à double spécificité, l'une dirigée vers la cellule-cible, l'autre vers l'un des haptènes définis ci-dessus.

## Revendications (Revendications pour l'(les) Etat(s) contractant(s) suivant(s) : ES)

1. Procédé de préparation d'un dérivé schématisé par la formule 1 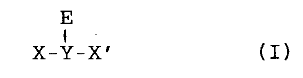 dans laquelle :
- X et X' sont des haptènes, c'est à dire des groupements organiques de masse inférieure à 1 500 daltons capables de se lier à un anticorps avec une constante de dissociation inférieure à 10⁻⁶ mole/l, caractérisés par leur propriété hydrophile définie par un coefficient de partage supérieur à 1 entre une phase aqueuse et un solvant organique en faveur de la phase aqueuse,
- Y est une chaîne de connexion permettant la liaison simultanée sur le même dérivé de formule l dau moins deux anticorps spécifiques desdits haptènes,
- E est un groupement effecteur, c'est-à-dire apte au marquage radioactif,
- étant entendu que ni X, ni X' ne peut être le benzyl EDTA ou un dérivé du benzyl EDTA, et que la chaîne de connexion ne peut pas être un polymère,
dans lequel on relie de manière stable les haptènes X et X' et le groupement effecteur E à l'aide de la chaîne de connexion Y.

2. Procédé selon la revendication 1 caractérisé en ce que le groupement effecteur E coïncide avec un des haptènes X ou X'.

3. Procédé selon l'une quelconque des revendications 1 et 2, caractérisé en ce que les haptènes sont des groupes chélatants.

4. Procédé selon l'une quelconque des revendications 1 à 3, caractérisé en ce que la chaîne de connexion est un peptide.

5. Procédé selon l'une quelconque des revendications 3 ou 4, caractérisé en ce que le groupe chélatant est un dérivé du DTPA.

6. Procédé selon l'une quelconque des revendications 1 à 5, caractérisé en ce que la chaîne de connexion comprend un groupement phénol.

7. Procédé selon la revendication 6, caractérisé en ce que la chaîne de connexion comprend de la tyrosine ou est constituée de tyrosine.

8. Procédé selon la revendication 1, caractérisé en ce que l'on prépare la N-α-DTPA-tyrosyl-N-ε-DTPA-lysine.

9. Procédé selon la revendication 1, caractérisé en ce que l'on prépare la N-α-N-ε-di-(DTPA-glycyl)-lysyl-tyrosine.

10. Procédé selon la revendication 1, caractérisé en ce que l'on prépare la N-α-N-ε-di-(histamine-succinyl-glycyl)-lysyl-tyrosine.

11. Procédé selon la revendication 1, caractérisé en ce que l'on prépare le N-α-acétyl,N-ε-DTPA,L-lysil-L-tyrosyl-N-ε-DTPA-L-lysyl-amide.

12. Procédé selon la revendication 1, caractérisé en ce que l'on prépare le N-a-acétyl, N-e-(histamine-succinyl-glycyl)-L-lysyl-L-tyrosyl-N-ε-(histamine-succinyl-glycyl)-L-lysyl-amide.

13. Kits diagnostiques ou thérapeutiques, dans lesquels on assemble au moins un des dérivés obtenus selon le procédé de l'une quelconque des revendications 1 à 12 et un anticorps ou fragment d'anticorps, notamment monoclonal reconnaissant un type cellulaire ou un tissu particulier, conjugué à un deuxième anticorps ou fragment d'anticorps reconnaissant un groupement hapténique dudit dérivé.

14. Procédé de préparation de réactifs immunologiques dans lequel on assemble :
- un conjugué composé d'un anticorps ou fragment d'anticorps notamment monoclonal ayant une affinité pour un type cellulaire particulier ou un constituant normal ou pathologique de l'organisme particulier, couplé à un anticorps ou fragment d'anticorps notamment monoclonal ayant une affinité pour un haptène donné, et
- une molécule synthétique comprenant au moins deux haptènes correspondant au conjugué ci-dessus et au moins un site, apte au marquage radioactif ou à fixer à un principe actif, liés de manière covalente,
caractérisé en ce que la molécule synthétique est un dérivé obtenu selon un procédé tel que défini à l'une' des revendications 1 à 12.

15. Les dérivés schématisés par la formule 1 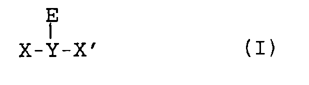 dans laquelle :
- X et X' sont des haptènes, c'est à dire des groupements organiques de masse inférieure à 1 500 daltons capables de se lier à un anticorps avec une constante de dissociation inférieure à 10⁻⁶ mole/l, caractérisés par leur propriété hydrophile définie par un coefficient de partage supérieur à 1 entre une phase aqueuse et un solvant organique en faveur de la phase aqueuse,
- Y est une chaîne de connexion permettant la liaison simultanée sur le même dérivé de formule l d'au moins deux anticorps spécifiques desdits haptènes,
- E est un groupement effecteur, c'est-à-dire apte au marquage radioactif,
- étant entendu que ni X, ni X' ne peut être le benzyl EDTA ou un dérivé du benzyl EDTA, et que la chaîne de connexion ne peut pas être un polymère,
pour leur application dans une méthode de diagnostic ou de traitement du corps humain ou animal.

16. Application des dérivés schématisés par la formule l 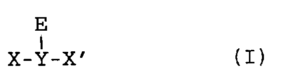 dans laquelle :
- X et X' sont des haptènes, c'est à dire des groupements organiques de masse inférieure à 1 500 daltons capables de se lier à un anticorps avec une constante de dissociation inférieure à 10⁻⁶ mole/l, caractérisés par leur propriété hydrophile définie par un coefficient de partage supérieur à 1 entre une phase aqueuse et un solvant organique en faveur de la phase aqueuse,
- Y est une chaîne de connexion permettant la liaison simultanée sur le même dérivé de formule l d'au' moins deux anticorps spécifiques desdits haptènes,
- E est un groupement effecteur, c' est-à-dire apte au marquage radioactif,
étant entendu que ni X, ni X' ne peut être le benzyl EDTA ou un dérivé du benzyl EDTA, et que la chaîne de connexion ne peut pas être un polymère,
à l'augmentation du contraste dans la visualisation d'une cellule-cible par utilisation conjointe avec un anticorps à double spécificité, l'une dirigée vers la cellule-cible, l'autre vers l'un des haptènes définis ci-dessus.

## Claims (Claims for the following Contracting State(s) : AT, BE, CH, DE, DK, FR, GB, GR, IT, LI, LU, NL, SE)

1. The derivatives schematized by formula I 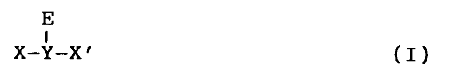 in which:
- X and X' are haptens, that is to say organic groups with a molecular weight of less than 1500 daltons capable of being bound to an antibody with a dissociation constant of less than 10⁻⁶ mole/l, characterized by their hydrophilic property defined by a partition coefficient of higher than 1 between an aqueous phase and an organic solvent in favour of the aqueous phase,
- Y is a connecting bridge allowing the simultaneous binding on the same derivative of formula I of at least two specific antibodies of the said haptens,
- E is an effector group, that is to say suitable for radioactive labelling,
- it being understood that neither X, nor X', can be EDTA benzyl or a derivative of EDTA benzyl, and that the connecting bridge cannot be a polymer.

2. The derivatives according to claim 1 characterized in that the effector group E coincides with one of the haptens X or X'.

3. The derivatives according to any one of claims 1 to 2, characterized in that the haptens are chelating groups.

4. The derivatives according to any one of claims 1 to 3, characterized in that the connecting bridge is a peptide.

5. The derivatives according to any one of claims 3 or 4, characterized in that the chelating group is a derivative of DTPA.

6. The derivatives according to any one of claims 1 to 5, characterized in that the connecting bridge contains a phenol group.

7. The derivatives according to claim 6, characterized in that the connecting bridge contains tyrosine or is constituted by tyrosine.

8. N-α-DTPA-tyrosyl-N-ε-DTPA-lysine.

9. N-α-N-ε-di-(DTPA-glycyl)-lysyl-tyrosine.

10. N-α-N-ε-di-(histamine-succinyl-glycyl)-lysyl-tyrosine.

11. N-α-acetyl,N-ε-DTPA,L-lysil-L-tyrosyl-N-ε-DTPA-L-lysyl-amide.

12. N-a-acetyl, N-ε-(histamine-succinyl-glycyl)-L-lysyl-L-tyrosyl-N-ε-(histamine-succinyl-glycyl)-L-lysyl-amide.

13. Diagnostic or therapeutic kits, characterized in that they contain at least one of the derivatives according to any one of claims 1 to 12 and an antibody or antibody fragment, in particular a monoclonal antibody recognizing a cellular type or a particular tissue, conjugated with a second antibody or antibody fragment recognizing a hapten group of the said derivative.

14. lmmunological reagents comprising:
- a conjugate composed of an antibody or antibody fragment in particular a monoclonal antibody having an affinity for a particular cellular type or a normal or pathological constituent of the particular organism, conjugated with an antibody or antibody fragment in particular a monoclonal antibody having an affinity for a given hapten, and
- a synthetic molecule containing at least two haptens corresponding to the above conjugate and at least one site, suitable for radioactive labelling or fixing to an active ingredient, bound in a covalent manner, characterized in that the synthetic molecule is a derivative defined in one of claims 1 to 12.

15. The derivatives schematized by formula I 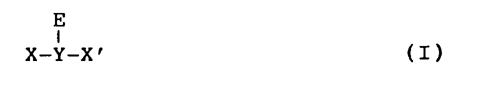 in which:
X and X' are haptens, that is to say organic groups with a molecular weight of less than 1500 daltons capable of being bound to an antibody with a dissociation constant of less than 10-⁶ molell, characterized by their hydrophilic property defined by a partition coefficient of higher than 1 between an aqueous phase and an organic solvent in favour of the aqueous phase,
- Y is a connecting bridge allowing the simultaneous binding on the same derivative of formula I of at least two specific antibodies of the said haptens,
E is an effector group, that is to say suitable for radioactive labelling,
- it being understood that neither X, nor X', can be EDTA benzyl or a derivative of EDTA benzyl, and that the connecting bridge cannot be a polymer,
for their use in a method for the diagnosis or treatment of humans or animals.

16. Use of the derivatives schematized by formula I 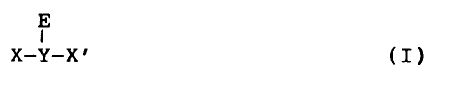 in which:
- X and X' are haptens, that is to say organic groups with a molecular weight of less than 1500 daltons capable of being bound to an antibody with a dissociation constant of less than 10⁻⁶ mole/l, characterized by their hydrophilic property defined by a partition coefficient of higher than 1 between an aqueous phase and an organic solvent in favour of the aqueous phase,
Y is a connecting bridge allowing the simultaneous binding on the same derivative of formula I of at least two specific antibodies of the said haptens,
E is an effector group, that is to say suitable for radioactive labelling,
it being understood that neither X, nor X', can be EDTA benzyl or a derivative of EDTA benzyl, and that the connecting bridge cannot be a polymer,
for the increase of the contrast in the visualization of a target cell by use in conjunction with an antibody with double specificity, one directed towards the target cell, the other towards one of the haptens defined above.

## Claims (Claims for the following Contracting State(s) : ES)

1. Preparation process for a derivative schematized by formula I 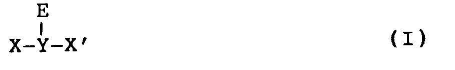 in which:
- X and X' are haptens, that is to say organic groups with a molecular weight of less than 1500 daltons capable of being bound to an antibody with a dissociation constant of less than 10⁻⁶ mole/l , characterized by their hydrophilic property defined by a partition coefficient of higher than 1 between an aqueous phase and an organic solvent in favour of the aqueous phase,
- Y is a connecting bridge allowing the simultaneous binding on the same derivative of formula I of at least two specific antibodies of the said haptens,
- E is an effector group, that is to say suitable for radioactive labelling,
- it being understood that neither X, nor X', can be EDTA benzyl or a derivative of EDTA benzyl, and that the connecting bridge cannot be a polymer,
in which the haptens X and X' and the effector group E are linked together in a stable manner using the connecting bridge Y

2. Process according to claim 1 characterized in that the effector group E coincides with one of the haptens X or X'.

3. Process according to any one of claims 1 and 2, characterized in that the haptens are chelating groups.

4. Process according to any one of claims 1 to 3, characterized in that the connecting bridge is a peptide.

5. Process according to any one of claims 3 or 4, characterized in that the chelating group is a derivative of DTPA.

6. Process according to any one of claims 1 to 5, characterized in that the connecting bridge contains a phenol group.

7. Process according to claim 6, characterized in that the connecting bridge contains tyrosine or is constituted by tyrosine.

8. Process according to claim 1, characterized in that N-a-DTPA-tyrosyl-N-s-DTPA-lysine is prepared.

9. Process according to claim 1, characterized in that N-α-N-ε-di-(DTPA-glycyl)-lysyl-tyrosine is prepared.

10. Process according to claim 1, characterized in that N-α-N-ε-di-(histamine-succinyl-glycyl)-lysyl-tyrosine is prepared.

11. Process according to claim 1, characterized in that N-α-acetyl, N-α-DTPA,L-lysil-L-tyrosyl-N-ε-DTPA-L-lysyl-amide is prepared.

12. Process according to claim 1, characterized in that N-a-acetyl, N-s-(histamine-succinyl-glycyl)-L-lysyl-L-tyrosyl-N-ε-(histamine-succinyl-glycyl)-L-lysyl-amide is prepared.

13. Diagnostic or therapeutic kits, in which at least one of the derivatives obtained according to the process of any one of claims 1 to 12 and an antibody or antibody fragment, in particular a monoclonal antibody recognizing a cellular type of a particular tissue, conjugated with a second antibody or antibody fragment recognizing a hapten group of the said derivative are assembled.

14. Preparation process for immunological reagents in which the following are assembled:
- a conjugate composed of an antibody or antibody fragment in particular a monoclonal antibody having an affinity for a particular cellular type or a normal or pathological constituent of the particular organism, coupled with an antibody or antibody fragment in particular a monoclonal antibody having an affinity for a given hapten, and
- a synthetic molecule containing at least two haptens corresponding to the above conjugate and at least one site, suitable for radioactive labelling or fixing to an active ingredient, bound in a covalent manner,
characterized in that the synthetic molecule is a derivative obtained according to a process as defined in one of claims 1 to 12.

15. The derivatives schematized by formula I 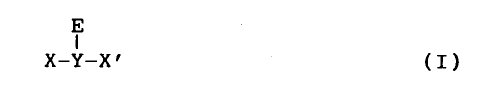 in which:
- X and X' are haptens, that is to say organic groups with a molecular weight of less than 1500 daltons capable of being bound to an antibody with a dissociation constant of less than 10-⁶ mole/l, characterized by their hydrophilic property defined by a partition coefficient of higher than 1 between an aqueous phase and an organic solvent in favour of the aqueous phase,
- Y is a connecting bridge allowing the simultaneous binding on the same derivative of formula I of at least two specific antibodies of the said haptens,
- E is an effector group, that is to say suitable for radioactive labelling,
- it being understood that neither X, nor X', can be EDTA benzyl or a derivative of EDTA benzyl, and that the connecting bridge cannot be a polymer,
for their use in a method for the diagnosis or treatment of humans or animals.

16. Use of the derivatives schematized by formula I 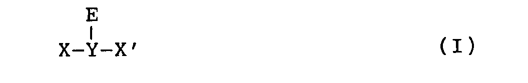 in which:
- X and X' are haptens, that is to say organic groups with a molecular weight of less than 1500 daltons capable of being bound to an antibody with a dissociation constant of less than 10-⁶ mole/l, characterized by their hydrophilic property defined by a partition coefficient of higher than 1 between an aqueous phase and an organic solvent in favour of the aqueous phase,
- Y is a connecting bridge allowing the simultaneous binding on the same derivative of formula I of at least two specific antibodies of the said haptens,
- E is an effector group, that is to say suitable for radioactive labelling,
- it being understood that neither X, nor X', can be EDTA benzyl or a derivative of EDTA benzyl, and that the connecting bridge cannot be a polymer,
for the increase of the contrast in the visualization of a target cell by use in conjunction with an antibody with double specificity, one directed towards the target cell, the other towards one of the haptens defined above.

## Patentansprüche (Patentansprüche für folgende(n) Vertragsstaat(en) : AT, BE, CH, DE, DK, FR, GB, GR, IT, LI, LU, NL, SE)

1. Derivate der Formel (I) 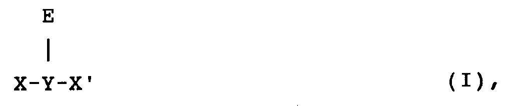 worin:
X und X' Haptene bedeuten, d.h. organische Gruppen mit einer Masse von weniger als 1500 Dalton, welche an einen Antikörper mit einer Dissoziationskonstante von weniger als 10-⁶ mol/I binden können, und welche durch ihre hydrophile Eigenschaft gekennzeichnet sind, die durch einen Verteilungskoeffizienten von mehr als 1 zwischen einer wässerigen Phase und einem organischen Lösungsmittel zugunsten der wässerigen Phase definiert ist;
Y eine Bindungskette darstellt, welche die gleichzeitige Bindung von zumindest zwei spezifischen Antikörpern der Haptene an dasselbe Derivat der Formel (I) ermöglicht;
E eine Effektor-Gruppe ist, d.h. für eine radioaktive Markerung geeignet ist;
mit der Maßgabe, daß weder X noch X' Benzyl-EDTA oder ein Derivat von Benzyl-EDTA sein kann, und die Bindungskette kein Polymer sein kann.

2. Derivate nach Anspruch 1, dadurch gekennzeichnet, daß die Effektor-Gruppe E mit einem der Haptene X oder X' koinzidiert.

3. Derivate nach Anspruch 1 oder 2, dadurch gekennzeichnet, daß die Haptene Chelatbildner-Gruppen sind.

4. Derivate nach einem der Ansprüche 1 bis 3, dadurch gekennzeichnet, daß die Bindungskette ein Peptid ist.

5. Derivate nach Anspruch 3 oder 4, dadurch gekennzeichnet, daß die Chelatbildner-Gruppe ein DTPA-Derivat ist.

6. Derivate nach einem der Ansprüche 1 bis 5, dadurch gekennzeichnet, daß die Bindungskette eine Phenol-Gruppe umfaßt.

7. Derivate nach Anspruch 6, dadurch gekennzeichnet, daß die Bindungskette Tyrosin umfaßt oder aus Tyrosin besteht.

8. N-α-DTPA-Tyrosyl-N-s-DTPA-lysin.

9. N-a-N-s-Di-(DTPA-glycyl)-lysyltyrosin.

10. N-a-N-c-Di-(histaminsuccinylglycyl)-Iysyltyrosin.

11. N-α-Acetyl-N-ε-DTPA-L-lysyl-L-tyrosyl-N-ε-DTPA-L-lysylamid.

12. N-α-Acetyl-N-ε-(histaminsuccinylglycyl)-L-lysyl-L-tyrosyl-N-ε-(histaminsuccinylglycyl)-L-lysylamid.

13. Diagnose- oder Therapie-Kits, dadurch gekennzeichnet, daß sie zumindest eines der Derivate nach einem der Ansprüche 1 bis 12 und einen, insbesondere monoklonalen, Antikörper oder ein Fragment eines Antikörpers, der einen Zelltyp oder ein bestimmtes Gewebe erkennen kann, konjugiert an einen zweiten Antikörper oder ein Fragment eines Antikörpers, der eine Hapten-Gruppe des Derivats erkennt, umfassen.

14. Immunologische Reagentien, welche umfassen:
ein Konjugat, bestehend das aus einem, insbesondere monoklonalen, Antikörper oder einem Fragment eines Antikörpers mit einer Affinität für einen bestimmten Zelltyp oder einen normalen oder pathologischen Bestandteil des betreffenden Organismus, gekoppelt mit einem, insbesondere monoklonalen, Antikörper oder einem Fragment eines Antikörpers mit einer Affinität für ein gegebenes Hapten, und
ein synthetisches Molekül mit zumindest zwei Haptenen, die dem obigen Konjugat entsprechen, und zumindest einer Stelle, die für eine radioaktive Markierung oder für eine Fixierung an einen aktiven Bestandteil geeignet ist, welche Haptene kovalent gebunden sind,
dadurch gekennzeichnet, daß das synthetische Molekül ein in einem der Ansprüche 1 bis 12 definiertes Derivat ist.

15. Derivate der Formel (I) 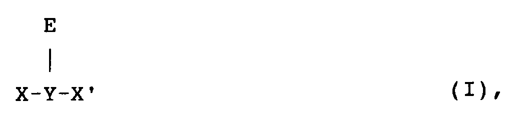 worin:
X und X' Haptene bedeuten, d.h. organische Gruppen mit einer Masse von weniger als 1500 Dalton, welche an einen Antikörper mit einer Dissoziationskonstante von weniger als 10-⁶ mol/l binden können, und welche durch ihre hydrophile Eigenschaft gekennzeichnet sind, die durch einen Verteilungskoeffizienten von mehr als 1 zwischen einer wässerigen Phase und einem organischen Lösungsmittel zugunsten der wässerigen Phase definiert ist;
Y eine Bindungskette darstellt, welche die gleichzeitige Bindung von zumindest zwei spezifischen Antikörpern der Haptene an dasselbe Derivat der Formel (I) ermöglicht;
E eine Effektor-Gruppe ist, d.h. für eine radioaktive Markierung geeignet ist;
mit der Maßgabe, daß weder X noch X' Benzyl-EDTA oder ein Derivat von Benzyl-EDTA sein kann, und die Bindungskette kein Polymer sein kann;
zur Verwendung in einem Diagnose- oder Behandlungsverfahren des Körpers von Menschen oder Tieren.

16. Verwendung von Derivaten der Formel (I) 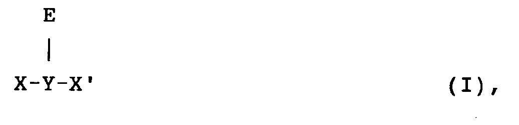 worin:
X und X' Heptene bedeuten, d.h. organische Gruppen mit einer Masse von weniger als 1500 Dalton, welche an einen Antikörper mit einer Dissoziationskonstante von weniger als 10-⁶ mol/l binden können, und welche durch ihre hydrophile Eigenschaft gekennzeichnet sind, die durch einen Verteilungskoeffizienten von mehr als 1 zwischen einer wässerigen Phase und einem organischen Lösungsmittel zugunsten der wässerigen Phase definiert ist;
Y eine Bindungskette darstellt, welche die gleichzeitige Bindung von zumindest zwei spezifischen Antikörpern der Haptene an demselben Derivat der Formel (I) ermöglicht;
E eine Effektor-Gruppe ist, d.h. für eine radioaktive Markierung geeignet ist;
mit der Maßgabe, daß weder X noch X' Benzyl-EDTA oder ein Derivat von Benzyl-EDTA sein kann, und die Bindungskette kein Polymer sein kann;
zur Erhöhung des Kontrasts bei der Visualisierung einer Zielzelle durch die gleichzeitige Verwendung mit einem Antikörper mit doppelter Spezifität, wobei die eine auf die Zielzelle und die andere auf eines der oben definierten Haptene gerichtet ist.

## Patentansprüche (Patentansprüche für folgende(n) Vertragsstaat(en) : ES)

1. Verfahren zur Herstellung eines Derivats der Formel (I) 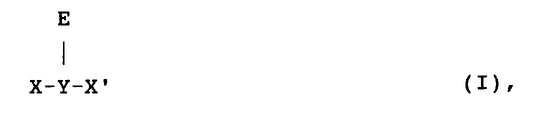 worin:
X und X' Haptene bedeuten, d.h. organische Gruppen mit einer Masse von weniger als 1500 Dalton, welche an einen Antikörper mit einer Dissoziationskonstante von weniger als 10-⁶ mol/I binden können, und welche durch ihre hydrophile Eigenschaft gekennzeichnet sind, die durch einen Verteilungskoeffizienten von mehr als 1 zwischen einer wässerigen Phase und einem organischen Lösungsmittel zugunsten der wässerigen Phase definiert ist;
Y eine Bindungskette darstellt, welche die gleichzeitige Bindung von zumindest zwei spezifischen Antikörpern der Haptene an dasselbe Derivat der Formel (I) ermöglicht;
E eine Effektor-Gruppe ist, d.h. für eine radioaktive Markerung geeignet ist;
mit der Maßgabe, daß weder X noch X' Benzyl-EDTA oder ein Derivat von Benzyl-EDTA sein kann, und die Bindungskette kein Polymer sein kann;
bei welchem Verfahren die Haptene X und X' und die Effektor-Gruppe E mit Hilfe der Bindungskette Y stabil gebunden werden.

2. Verfahren nach Anspruch 1, dadurch gekennzeichnet, daß die Effektor-Gruppe E mit einem der Haptene X oder X' koinzidiert.

3. Verfahren nach Anspruch 1 oder 2, dadurch gekennzeichnet, daß die Haptene Chelatbildner-Gruppen sind.

4. Verfahren nach einem der Ansprüche 1 bis 3, dadurch gekennzeichnet, daß die Bindungskette ein Peptid ist.

5. Verfahren nach Anspruch 3 oder 4, dadurch gekennzeichnet, daß die Chelatbildner-Gruppe ein DTPA-Derivat ist.

6. Verfahren nach einem der Ansprüche 1 bis 5, dadurch gekennzeichnet, daß die Bindungskette eine Phenol-Gruppe umfaßt.

7. Verfahren nach Anspruch 6, dadurch gekennzeichnet, daß die Bindungskette Tyrosin umfaßt oder aus Tyrosin besteht.

8. Verfahren nach Anspruch 1, dadurch gekennzeichnet, daß N-α-DTPA-Tyrosyl-N-s-DTPA-lysin hergestellt wird.

9. Verfahren nach Anspruch 1, dadurch gekennzeichnet, daß N-α-N-ε-Di-(DTPA-glycyl)-lysyltyrosin hergestellt wird.

10. Verfahren nach Anspruch 1, dadurch gekennzeichnet, daß N-α-N-ε-Di-(histaminsuccinylglycyl)-lysyltyrosin hergestellt wird.

11. Verfahren nach Anspruch 1, dadurch gekennzeichnet, daß N-α-Acetyl-N-ε-DTPA-L-lysyl-L-tyrosyl-N-ε-DTPA-L-lysylamid hergestellt wird.

12. Verfahren nach Anspruch 1, dadurch gekennzeichnet, daß N-a-Acetyl-N-r-(histaminsuccinylglycyl)-L-lysyl-L-tyrosyl-N-ε-(histaminsuccinylglycyl)-L-lysylamid hergestellt wird.

13. Diagnose- oder Therapie-Kits, dadurch gekennzeichnet, daß sie zumindest eines der nach einem der Ansprüche 1 bis 12 erhaltenen Derivate und einen, insbesondere monoklonalen, Antikörper oder ein Fragment eines Antikörpers, der einen Zelltyp oder ein bestimmtes Gewebe erkennen kann, konjugiert an einen zweiten Antikörper oder ein Fragment eines Antikörpers, der eine Hapten-Gruppe des Derivats erkennt, umfassen.

14. Verfahren zur Herstellung immunologischer Reagentien, bei welchem vereinigt werden:
ein Konjugat, bestehend das aus einem, insbesondere monoklonalen, Antikörper oder einem Fragment eines Antikörpers mit einer Affinität für einen bestimmten Zelltyp oder einen normalen oder pathologischen Bestandteil des betreffenden Organismus, gekoppelt mit einem, insbesondere monoklonalen, Antikörper oder einem Fragment eines Antikörpers mit einer Affinität für ein gegebenes Hapten, und
ein synthetisches Molekül mit zumindest zwei Haptenen, die dem obigen Konjugat entsprechen, und zumindest einer Stelle, die für eine radioaktive Markerung oder für eine Fixierung an einen aktiven Bestandteil geeignet ist, welche Haptene kovalent gebunden sind,
dadurch gekennzeichnet, daß das synthetische Molekül ein Derivat ist, das nach einem wie in einem der Ansprüche 1 bis 12 definierten Verfahren erhalten wird.

15. Derivate der Formel (I) 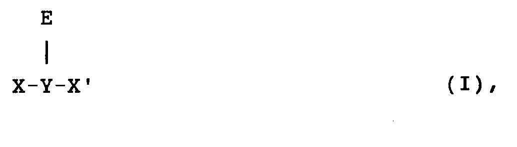 worin:
X und X' Haptene bedeuten, d.h. organische Gruppen mit einer Masse von weniger als 1500 Dalton, welche an einen Antikörper mit einer Dissoziationskonstante von weniger als 10-⁶ mol/l binden können, und welche durch ihre hydrophile Eigenschaft gekennzeichnet sind, die durch einen Verteilungskoeffizienten von mehr als 1 zwischen einer wässerigen Phase und einem organischen Lösungsmittel zugunsten der wässerigen Phase definiert ist;
Y eine Bindungskette darstellt, welche die gleichzeitige Bindung von zumindest zwei spezifischen Antikörpern der Haptene an dasselbe Derivat der Formel (I) ermöglicht;
E eine Effektor-Gruppe ist, d.h. für eine radioaktive Markierung geeignet ist; mit der Maßgabe, daß weder X noch X' Benzyl-EDTA oder ein Derivat von Benzyl-EDTA sein kann, und die Bindungskette kein Polymer sein kann,
zur Verwendung in einem Diagnose- oder Behandlungsverfahren des Körpers von Menschen oder Tieren.

16. Verwendung von Derivaten der Formel (I) 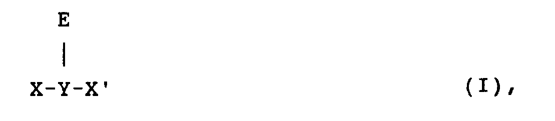 worin:
X und X' Heptene bedeuten, d.h. organische Gruppen mit einer Masse von weniger als 1500 Dalton, welche an einen Antikörper mit einer Dissoziationskonstante von weniger als 10-⁶ mol/l binden können, und welche durch ihre hydrophile Eigenschaft gekennzeichnet sind, die durch einen Verteilungskoeffizienten von mehr als 1 zwischen einer wässerigen Phase und einem organischen Lösungsmittel zugunsten der wässerigen Phase definiert ist;
Y eine Bindungskette darstellt, welche die gleichzeitige Bindung von zumindest zwei spezifischen Antikörpern der Haptene an dasselbe Derivat der Formel (I) ermöglicht;
E eine Effektor-Gruppe ist, d.h. für eine radioaktive Markierung geeignet ist;
mit der Maßgabe, daß weder X noch X' Benzyl-EDTA oder ein Derivat von Benzyl-EDTA sein kann, und die Bindungskette kein Polymer sein kann;
zur Erhöhung des Kontrasts bei der Visualisierung einer Zielzelle durch die gleichzeitige Verwendung mit einem Antikörper mit doppelter Spezifität, wobei die eine auf die Zielzelle und die andere auf eines der oben definierten Haptene gerichtet ist.
